(19) **Europäisches Patentamt / European Patent Office / Office européen des brevets**

(11) **EP 4 376 703 B1**

(12) **EUROPEAN PATENT SPECIFICATION**

(45) Date of publication and mention
of the grant of the patent:
**24.12.2025 Bulletin 2025/52**

(21) Application number: **22754418.6**

(22) Date of filing: **26.07.2022**

(51) International Patent Classification (IPC):
**A61B 5/00** *(2006.01)* **A61B 5/374** *(2021.01)*

(52) Cooperative Patent Classification (CPC):
**A61B 5/4821; A61B 5/374; A61B 5/7207; A61B 5/726**

(86) International application number:
**PCT/EP2022/070886**

(87) International publication number:
**WO 2023/006712 (02.02.2023 Gazette 2023/05)**

(54) **COMPUTER-IMPLEMENTED METHOD, DEVICE AND COMPUTER-READABLE MEDIUM FOR ASSISTING A GENERAL ANESTHESIA OF A SUBJECT**

COMPUTERIMPLEMENTIERTES VERFAHREN, GERÄT UND COMPUTERLESBARES SPEICHERMEDIUM ZUR UNTERSTÜTZUNG EINER ALLGEMEINEN NARKOSE EINES PATIENTEN

PROCÉDÉ MIS EN OEUVRE PAR ORDINATEUR, DISPOSITIF ET SUPPORT D'ENREGISTREMENT LISIBLE POUR ASSISTER UNE ANESTHÉSIE GÉNÉRALE D'UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2021 EP 21306053**

(43) Date of publication of application:
**05.06.2024 Bulletin 2024/23**

(73) Proprietors:
• **PARIS SCIENCES ET LETTRES**
**75006 Paris (FR)**
• **Centre National de la Recherche Scientifique (CNRS)**
**75016 Paris (FR)**
• **Institut National de la Santé et de la Recherche Médicale**
**75013 Paris (FR)**
• **Assistance Publique - Hôpitaux de Paris**
**75012 Paris (FR)**

(72) Inventors:
• **HOLCMAN, David**
**75003 PARIS (FR)**
• **DORA, Matteo**
**75011 PARIS (FR)**

• **SUN, Christophe**
**75014 PARIS (FR)**

(74) Representative: **Regimbeau**
**20, rue de Chazelles**
**75847 Paris Cedex 17 (FR)**

(56) References cited:
**WO-A1-2021/081504**

• **DORA MATTEO ET AL: "Adaptive single-channel EEG artifact removal with applications to clinical monitoring", MEDRXIV, 26 October 2021 (2021-10-26), XP055873292, Retrieved from the Internet <URL:https://www.medrxiv.org/content/10.1101/2021.10.19.21265197v1.full.pdf> [retrieved on 20211215], DOI: 10.1101/2021.10.19.21265197**
• **CARTAILLER JÉRÔME ET AL: "Alpha rhythm collapse predicts iso-electric suppressions during anesthesia", vol. 2, no. 1, 1 December 2019 (2019-12-01), XP055873174, Retrieved from the Internet <URL:https://www.nature.com/articles/s42003-019-0575-3.pdf> [retrieved on 20220111], DOI: 10.1038/s42003-019-0575-3**

**Description**

**FIELD OF THE INVENTION**

The present invention relates to the field of method for assisting a general anesthesia of a subject and in particular electroencephalogram (EEG) signal analysis in the specific context of general anesthesia (GA).

**BACKGROUND OF THE INVENTION**

General anesthesia is a medically induced coma with loss of protective reflexes, resulting from the administration of one or more anesthetic agents. It is carried out to allow medical procedures that would otherwise be intolerably painful for the subject, or where the nature of the procedure itself precludes the patient being awake. In order to be in control of a general anesthesia, the literature mainly provides a first approach consisting in signal processing to analyze real time events such as electroencephalogram (EEG) signal or a second approach consisting in measuring and estimating the effect of parameters such as the age, the arterial pressure, etc.

Monitoring a general anesthesia is currently based on the use of EEG, which is easy and inexpensive to acquire. EEG is the benchmark for monitoring intraoperative brain activity.

This monitoring is mainly focused on the depth of anesthesia (DoA), defined as the degree of central nervous system depression produced by anesthetics. Commercial DoA monitors can reveal various characteristics determined from the EEG signal, such as percentage of fast and slow wave or the degree of wave synchronization.

Various frequency bands can be extracted and also transient patterns such as iso-electrical suppressions (IES) that consist of periods of iso-electric (flat) activity lasting from few seconds to several minutes. These suppressions appear in several pathological conditions such as epileptic encephalopathies, drug intoxications, comatose or brain death. They are also associated with postoperative sleep disorder, the emergence of delirium, and an increased mortality in sedated critically ill persons. IES can also appear when the concentrations of anesthetics increase.

It is therefore a recommendation to avoid anesthetic drug-related IES during anesthesia.

If commercial DoA monitors can detect IES, those tools cannot predict the occurrence of IES.

More generally, there is no robust method to anticipate and prevent IES, and no method to predict the sensitivity of a subject to general anesthesia.

Being able to predict IES sufficiently in advance with a high accuracy make possible to act on the doses of anesthetic administered, for example by reducing them.

There is thus a need to provide for predictive information on the sensitivity of a subject to general anesthesia and the risk of IES due to general anesthesia.

Further background art is disclosed in document WO 2021/081504 A1, and Cartailler, J., Parutto, P., Touchard, C. et al. Alpha rhythm collapse predicts iso-electric suppressions during anesthesia. Commun Biol 2, 327 (2019). https://doi.org/10.1038/s42003-019-0575-3.

**BRIEF DESCRIPTION OF THE INVENTION**

It is an object of this invention to provide a method for estimating a sensitivity of a subject to general anesthesia or sedation that allows to act on the doses of anesthetic administered.

To that end, there is provided a computer-implemented method for assisting a general anesthesia or a sedation of a subject, comprising :

- receiving an electroencephalogram EEG signal of a subject,
- processing the EEG signal comprising $\alpha$-bands for

   -- defining for each instant of a plurality of instants, a part of the signal comprised in a time window comprising the instant, the part of the signal being related to the instant,
   -- determining for each instant, a distribution of a power of the part of the signal as a function of the frequency,
   -- determining for each instant a maximum power frequency as a frequency of maximum power in the distribution, the maximum power frequency being related to the instant,

- detecting a reference instant, so that the frequency of maximal power takes higher values before the reference instant than after,
- detecting in a time frame following the first instant, an alpha instant corresponding to a first occurrence of a suppression of an $\alpha$-band,

- estimating a first parameter as the duration between the reference instant and the alpha instant, said first parameter relating to a sensitivity of the subject to a general anesthesia.

First the alpha duration can be extracted in the first minutes following the loss of consciousness of the general anesthesia, that is significantly before the administration of doses of anesthetic for the maintenance phase.

Second, the alpha duration carries relevant information with regards to iso-electrical suppressions (IES) the subject may experience in the close future.

This method thus provides predictive information on the sensitivity of a subject to general anesthesia sufficiently in advance to possibly modify doses of anesthetic to be administered.

Such method is advantageously and optionally completed by the following features taken singly or in combination:

- detecting time spans in the signal, a suppression of an $\alpha$-band extending on each time span,
- estimating a second parameter as a rate of increase of a proportion of the time spans in the signal, said second parameter relating to a sensitivity of the subject to a general anesthesia or a sedation;
- detecting in a time frame following the reference instant, an iso-electric instant of a first occurrence of an iso-electric suppression,
- estimating a third parameter as the duration between the reference instant and the iso-electric instant, said third parameter relating to a sensitivity of the subject to a general anesthesia or a sedation;
- determining an induction instant following a predetermined induction duration after the reference instant,
- determining a final instant following a predetermined final duration after the induction instant,
- if the alpha instant is before the induction instant and the iso-electric instant, determining at the alpha instant the first parameter,
- if the iso-electric instant is before the induction instant and the alpha instant, determining at the iso-electric instant the third parameter,
- determining a first estimate of the second parameter at the induction instant,
- determining at the final instant, a second estimate of the second parameter, the first parameter if the alpha instant is before the final instant and the third parameter if the iso-electric instant is before the final instant;
- detecting the reference instant comprises

--fitting a curve of the frequency of maximal power as a function of time using a sigmoid function,
-- determining a range of values and a final value of the sigmoid function,

-- determining the reference instant as an instant when the sigmoid function reaches an intermediate value, a difference between the intermediate value and the final value being equal to 5% of the range of values;

- detecting an artifact signal within the signal,
- determining a ground signal using a portion of the signal immediately before or after the artifact,
- decomposing the artifact signal and respectively the ground signal using a discrete wavelet transform so as to determine, for each level of the transform an artifact projection of the artifact signal and respectively a ground projection of the ground signal, the artifact projection and respectively the ground projection being a projection of the artifact signal respectively the ground signal on a basis of the discrete wavelet transform,
- for each level m of the transform determining an artifact cumulative density function $F_m^{(art)}(x) = \frac{1}{N_m} \sum_{n=1}^{N_m} 1_{\left|w_{m,n}^{(art)}\right|<x}$ where $N_m$ is the total number of coefficients of the artifact projection at level m, $\left|w_{m,n}^{(art)}\right|$ is the absolute value of a n-th coefficient of the artifact projection at level m, and $1_{\left|w_{m,n}^{(art)}\right|<x}$ is an indicator function that returns the value 1 if $\left|w_{m,n}^{(art)}\right| < x$ and else the value 0,

- for each level m of the transform determining a ground cumulative density function $F_m^{(ref)}(x) = \frac{1}{N_m} \sum_{n=1}^{N_m} 1_{\left|w_{m,n}^{(ref)}\right|<x}$ where $N_m$ is the total number of coefficients of the artifact projection at level m, $\left|w_{m,n}^{(ref)}\right|$ is the absolute value of a n-th coefficient of the artifact projection at level m, and $1_{\left|w_{m,n}^{(ref)}\right|<x}$ is an indicator function that returns the value 1 if $\left|w_{m,n}^{(ref)}\right| < x$ and else the value 0,

- for each level m and for each artifact coefficient $w_{m,n}^{(art)}$ determining a corrected coefficient $w_{m,n}^{(corr)}$ of a corrected projection, the artifact coefficient and the corrected coefficient being similarly indexed, following the sub-steps:

-- determining the value $F_m^{(art)}\left(\left|w_{m,n}^{(art)}\right|\right)$ ,

-- determining an intermediate value $x$ verifying $F_m^{(ref)}(x) = F_m^{(art)}\left(\left|w_{m,n}^{(art)}\right|\right)$ ,

- setting the corrected coefficient $w_{m,n}^{(corr)}$ equal to $w_{m,n}^{(art)} \times min\left\{1, \frac{x}{\left|w_{m,n}^{(art)}\right|}\right\}$ ,

- determining a corrected signal using the discrete wavelet transform based on the corrected projection,
- replacing in the signal the artifact signal by the corrected signal;
- a determination of a minimum quantity of anesthetic to inject so that the subject remains in general anesthesia, the determination being based on the first parameter.

It is also an object of this invention to provide a general anesthesia or sedation assisting device comprising a processor configured for implementing the computer implemented method as described above.

It is another object of this invention to provide a computer-readable medium comprising computer-executable instructions embodied which, when executed by a computer, cause the computer to carry out the as described above.

The invention is defined by the appended claims 1-9.

## BRIEF DESCRIPTION OF THE DRAWINGS

Further features and advantages of the invention will be apparent from the following description, based on the appended drawings wherein:

[Fig. 1] represents a general anesthesia or sedation assisting device according to an embodiment of the invention;

[Fig. 2] represents an electroencephalogram signal;

[Fig. 3] represents a spectrogram of an electroencephalogram signal;

[Fig. 4] represents a frequency of maximum power $f_{max}(t)$ of an electroencephalogram signal as a function of time;

[Fig. 5] represents a curve fitting of the frequency of maximum power $f_{max}(t)$ of figure 4;

[Fig. 6] represents an electroencephalogram signal comprising suppressions of an $\alpha$-band and iso-electric suppressions;

[Fig. 7] represents a fraction of the signal occupied by suppressions of an $\alpha$-band, the fraction being a function of time;

[Fig. 8] represents ROC curves;

[Fig. 9] represents a decision tree method;

[Fig. 10] represents artifacts in an electroencephalogram signal;

[Fig. 11] represents a cumulative function defined through a wavelet decomposition of an electroencephalogram signal;

[Fig. 12] represents a separation of two groups of persons with regards of parameters extracted from an electroencephalogram signal.

Reference signs that are identical from one figure to another one designates the same element, or elements fulfilling the same function.

## DETAILED DESCRIPTION

*General anesthesia or sedation assisting device*

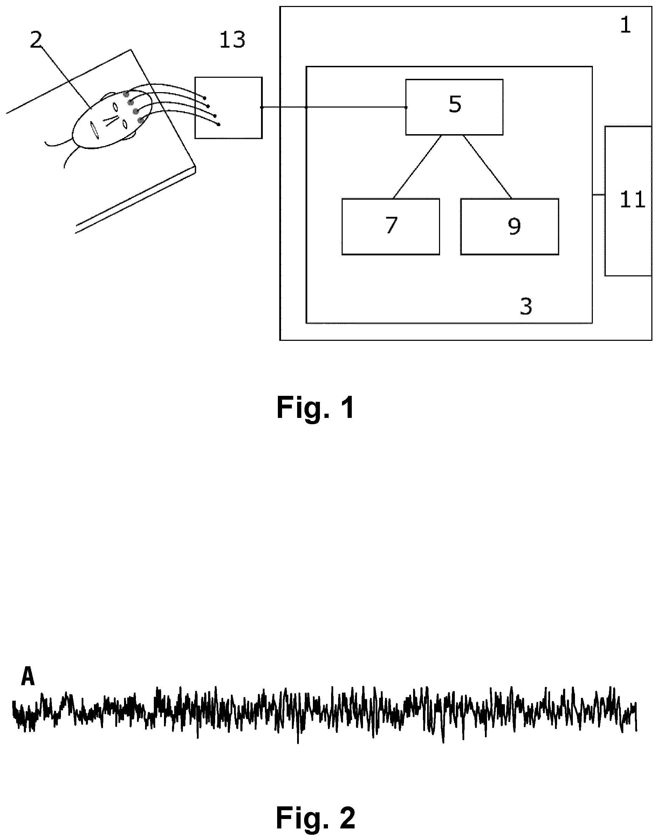

In reference to figure 1, a general anesthesia or sedation assisting device is now presented.

According to the invention, the terms "subject" and "patient" are used interchangeably herein and refer to a mammal or several mammals in need of a general anesthesia or a sedation procedure. Subjects are preferably humans or a person.

A person 2 carries electrodes of an electroencephalogram EEG acquisition device 13. The acquisition device 13 is configured to produce an electroencephalogram signal from the person being tested. The acquisition device 13 is connected to a general anesthesia or sedation assisting device 1 that comprises a processor 3 configured to receive and process an electroencephalogram EEG signal.

*Frequency analysis*

The processor 3 comprises a frequency analyzer unit 5 configured to receive the electroencephalogram EEG signal and extract frequency parameters related to the signal. In particular, the frequency analyzer 5 is configured to define a sliding time window and determine a truncated signal equal to the EEG signal within the sliding time window and equal to zero outside the sliding time window. The frequency analyzer is configured to determine a distribution of a power of the truncated signal as a function of the frequency and extract a frequency of maximum power $f_{max}$. As the time window is centered on an instant, the frequency of maximum power corresponds to an instant. The frequency analyzer 5 can thus produce a spectrogram of the EEG signal and extracts the frequency of maximum power $f_{max}(t)$ of the signal as a function of time.

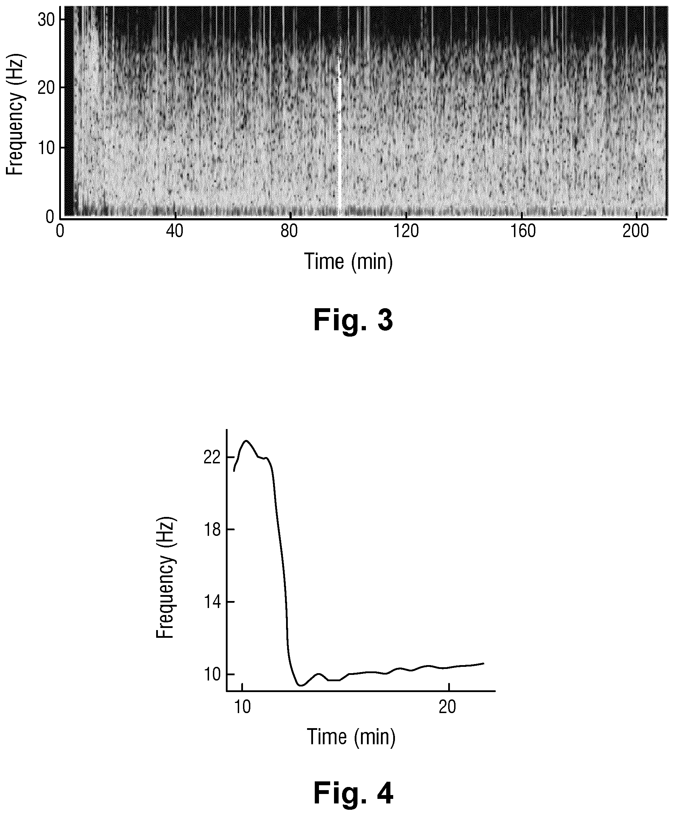

Figure 3 illustrates an example of a spectrogram of an EEG signal.

Figure 4 illustrates an example of the frequency of maximum power $f_{max}(t)$ of the signal as a function of time.

The processor 3 is configured to detect a reference instant, so that a function of time, for instance the frequency of maximal power $f_{max}(t)$, takes higher values before the reference instant than after.

*EEG pattern : suppression of an $\alpha$-band and iso-electric suppression*

The processor comprises also a memory 7 in which particular EEG patterns are recorded or predetermined threshold related to particular EEG patterns. In particular EEG patterns without an $\alpha$-band and EEG patterns of iso-electric suppressions are recorded or predetermined threshold related to particular EEG patterns without an $\alpha$-band and EEG patterns of iso-electric suppressions are recorded.

The processor 3 is configured to identify a suppression of an $\alpha$-band, further named $\alpha S$, within the signal and timestamp the suppression.

For this purpose, different methods may be implemented by the processor 3.

A first option consists in filtering the signal in the alpha band and checking that the amplitude is below a predetermined threshold related to particular EEG patterns without an $\alpha$-band and further in filtering the signal in the delta band and checking that the filtered signal presents a power above a predetermined power.

A second option consists in comparing the signal to particular EEG patterns without an $\alpha$-band and to particular EEG patterns of iso-electric suppressions and according to the similarities detected deciding if the signal compared corresponds to a suppression of an $\alpha$-band.

Simple indicators and simple filtering tools very common in the field are sufficient to make this identification of a suppression of an $\alpha$-band within the signal and timestamp of the suppression.

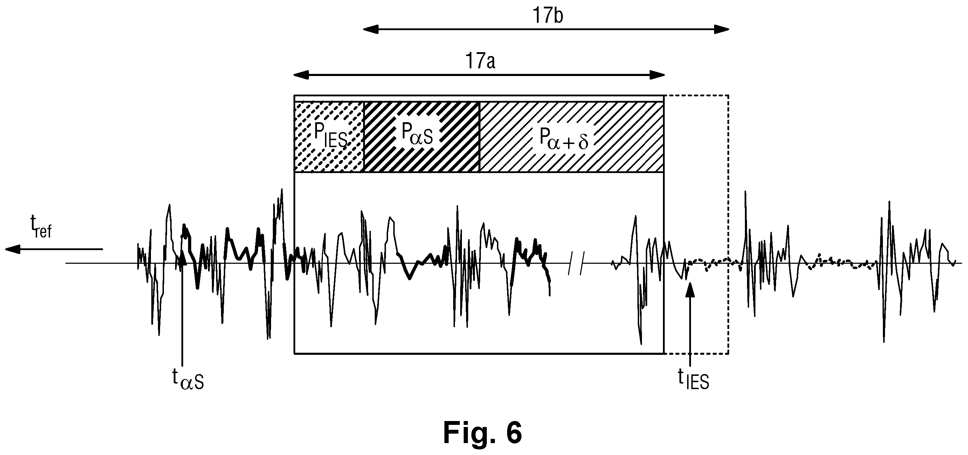

Figure 6 illustrates an example of an EEG signal. Only a part of the EEG signal is illustrated on Figure 6. The processor is configured to detect suppressions of an $\alpha$-band within the signal. The first suppression is illustrated on figure 6 and occurs at the alpha instant $t_{\alpha S}$.

The processor 3 may optionally be further configured to detect time spans in the signal, a suppression of an $\alpha$-band or $\alpha S$ extending on each time span. The processor is then configured to estimate a rate of increase of a proportion of the time spans in the signal. In other words, the processor defines sliding time windows in the signal and estimate the fraction of the time window that is occupied by the time spans. The duration of the sliding time window may be chosen equal to 240 seconds. The analysis of the sliding time window may be configured to analyze the content of time windows temporally separated with a delay of 1 second, so that two successive time windows present an overlap of 239 seconds.

Figure 6 illustrates a first time window 17a and a second time window 17b, the second time window 17b starting later than the first time window 17a. Time windows 17a and 17b present an overlap. Within the first time window 17a, the processor counts the time spans and estimates the fraction $P_{\alpha S}$ of the first time window 17a occupied by the time spans.

The time windows may also be occupied by periods of iso-electric suppressions and periods comprising Delta band in the range [0-4] Hz and an Alpha band in the range [8-14] Hz. Figure 6 illustrates a possible composition of the first time window 17a, that comprises :

- a fraction $P_{\alpha S}$ of suppression of an $\alpha$-band,
- a fraction $P_{IES}$ of iso-electric suppression,
- a fraction $P_{\alpha+\delta}$ of Delta band and Alpha band.

As the time window is centered on an instant, the fraction corresponds to an instant. The processor 3 can thus produce a fraction of the time window that is occupied by the time spans as a function of time. The processor 3 is also configured to determine a rate of increase of this fraction, or, put in another way, a proportion of the time spans in the signal.

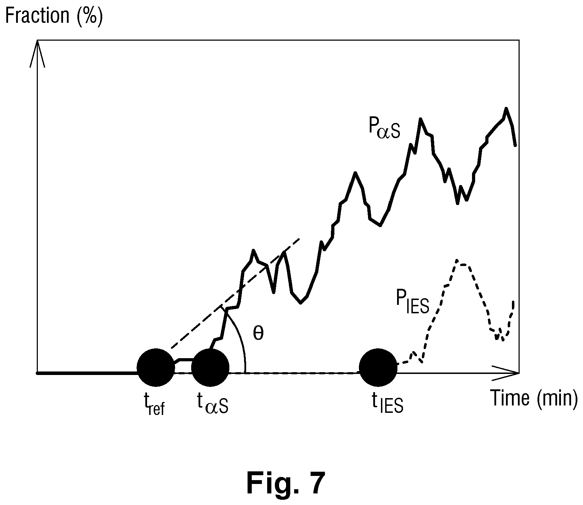

Figure 7 illustrates an example of a determination of a rate of increase of the fraction $P_{\alpha S}$ of suppression of an $\alpha$-band. The instants $t_{ref}$ and $t_{\alpha S}$ are represented on the abscises axis. On the ordinate axis, two fractions are represented: the fraction $P_{\alpha S}$ of suppression of an $\alpha$-band and the fraction $P_{IES}$ of iso-electric suppression.

$P_{\alpha S}$ is equal to zero for a time window temporally located before $t_{\alpha S}$ and takes positive values for a time window that comprises $t_{\alpha S}$ or that is temporally located after $t_{\alpha S}$. As more suppressions of an $\alpha$-band occur in the signal, $P_{\alpha S}$ takes higher values. The curve of $P_{\alpha S}$ as a function of time can be linearly fitted by the processor so as to extract a rate of increase $\tan(\theta)$ or equivalently a slope a. The processor 3 is configured to identify an iso-electric suppression, further named IES, within the signal and timestamp the suppression.

A first iso-electric suppression is illustrated on figure 6 and occurs at the iso-electric instant $t_{IES}$.

*Sigmoid Function*

A model of a sigmoid function is recorded in the memory 7 as follows

$$S(t) = \frac{a}{1 + exp\big(c(t - t_0)\big)} + b$$

where a, b, c and $t_0$ are parameters to be estimated.

The processor 3 is configured to fit a curve C(t) as a function of time using the sigmoid function. Parameters a, b, c and $t_0$ are found in the process of fitting as parameters minimizing a quantity $\|S(t) - C(t)\|_{L^2([0,T_0])}$ , or put in another form :

$$\{\hat{a}, \hat{b}, \hat{c}, \hat{t_0}\} = argmin_{a,b,c,t_0} \|S(t) - C(t)\|_{L^2([0,T_0])}$$

In particular, the curve C(t) can be chosen as the frequency of maximum power of the signal $f_{max}(t)$. Such a fitting procedure allows to determine the portion of the spectrogram, where the beta band disappears and the alpha bands emerges, involving the [8-30] Hz frequencies.

The processor 3 is configured to determine an initial value of the sigmoid function $S_{max} = a + b$, a final value of the sigmoid function $S_{min} = a$ and a range of values of the sigmoid function $S_{max} - S_{min} = b$.

The processor 3 is configured to determine an instant t* when the sigmoid function reaches an intermediate value, a difference between the intermediate value and the final value being equal to 5% of the range of values:

$$t * \frac{1}{c} ln\left(\frac{1}{0.05} - 1\right) + t_0.$$

Such an instant t* may be used as a reference instant, so that the sigmoid function takes higher values before the reference instant t* than after.

Other tools that do not involve a sigmoid function may be implemented to determine a reference instant based on the provided maximum power frequency as a function of time, so that the maximum power frequency as a function of time takes higher values before the reference instant than after. For instance, the person skilled in the art may use:

1- an algebraic function of the type $a + \frac{1}{(b+c\times t^\alpha)}$ or an exponential function of the type $e^{b(t-t0)}$ - both function of the variable t ; a,b,c, $\alpha$ and $t_0$ being parameters to be estimated - instead of the sigmoid function as described above and following similar steps, or

2-a relative power of alpha band computed with respect to the band [0.1-45] Hz and an estimation of the first time to a threshold.

The processor comprises also a calculator unit 9 configured to determine a first parameter as a duration between a reference instant and an alpha instant, a first occurrence of a suppression of an $\alpha$-band occurring at the alpha instant.

The calculator unit is also configured to determine a third parameter as a duration between a reference instant and an iso-electric instant, a first occurrence of a suppression of an iso-electric suppression occurring at the iso-electric instant.

*Computer-implemented method for assisting a general anesthesia or a sedation of a person*

The general anesthesia or sedation assisting device presented above may be used to implement a computer-implemented method for assisting a general anesthesia or a sedation of a person.

In a first step, an electroencephalogram EEG signal of a person is received by the assisting device. The EEG signal can be transferred from the acquisition device to the assisting device in real time or with a delay. The EEG signal may also be acquired by another acquisition device and later transferred to the assisting device.

Figure 2 illustrates an example of an EEG signal acquired through one of the frontal electrodes of an acquisition device.

The EEG signal is acquired on a person initially conscious that is put into an artificial coma. Before the induction of general anesthesia or sedation, the spectrogram of the EEG can contain a Beta band in the range [14-30] Hz, that characterizes a normal conscious awakening state. The person loses its consciousness at a reference instant $t_{ref}$ called LOC that stands for Loss of Consciousness. After the reference instant $t_{ref}$, two main bands appear at low rhythms: a Delta band in the range [0-4] Hz and an Alpha band in the range [8-14] Hz. The LOC may be defined as the instant when the dominant brain state switch from a Beta band to an Alpha band.

The EEG signal comprises $\alpha$-bands.

In a second step, the assisting device processes the EEG signal, extracts the frequency of maximum power $f_{max}$ (t) of the signal as a function of time, and detects the reference instant $t_{ref}$ defined in the following manner: the maximum power frequency as a function of time takes higher values before the reference instant than after. The assisting device also detects in a time frame following the reference instant, an alpha instant corresponding to a first occurrence of a suppression of an $\alpha$-band. Thanks to EEG patterns without an $\alpha$-band recorded in the memory, the processor establishes the alpha instant.

For example, a first suppression of an $\alpha$-band is illustrated on figure 6 and occurs at the alpha instant $t_{\alpha S}$.

In a third step, the assisting device estimates a first parameter as the duration between the reference instant and the alpha instant. This first parameter relates to a sensitivity of the person to a general anesthesia or sedation.

More specifically, the shorter the duration, the more sensitive is the person to a general anesthesia or sedation.

Following this method, one can determine a parameter relating to the sensitivity of the monitored person to general anesthesia or sedation, as soon as a suppression of an $\alpha$-band occurs. This can occur less than 10 minutes following the loss of consciousness, and enables to obtain relevant information sooner than in the prior art.

In the case where the EEG signal does not comprise any suppression of the $\alpha$-band, one can only find a minimal bound on the duration between the reference instant and the alpha instant. It is still possible to estimate the duration and retrieve a relevant information for this person who is in this case appear with no sensitivity or a low sensitivity to general anesthesia or sedation.

Following the estimation of the first parameter, a minimum quantity of anesthetic to inject so that the subject remains in general anesthesia or sedation may be determined based on the first parameter.

The computer-implemented method for assisting a general anesthesia or a sedation of a person may optionally comprise the following steps:

- detecting time spans in the signal, a suppression of an $\alpha$-band extending on each time span,
- estimating a second parameter as a rate of increase of a proportion of the time spans in the signal, said second parameter relating to a sensitivity of the person to a general anesthesia or sedation.

More specifically, the greater the rate of increase, the more sensitive the person is to general anesthesia or sedation.

The rate of increase corresponds to the term $\tan(\theta)$ or the slope a or described previously with regards to the linear fit of the curve of $P_{\alpha S}$ in figure 7.

In the case where the EEG signal does not comprise any suppression of the $\alpha$-band, one can estimate the second parameter as equal to zero.

Following this first option, one can determine a parameter relating to the sensitivity of the monitored person to general anesthesia or sedation, as soon as a plurality of suppressions of an $\alpha$-band have taken place. One can set a specific duration or induction duration about 10 minutes following the loss of consciousness, and obtain the second parameter relevant with regards of the sensitivity sooner than in the prior art.

The computer-implemented method for assisting a general anesthesia or a sedation of a person may optionally comprise the following steps

- detecting in a time frame following the reference instant, an iso-electric instant of a first occurrence of an iso-electric suppression,
- estimating a third parameter as the duration between the reference instant and the iso-electric instant, said third parameter relating to a sensitivity of the person to a general anesthesia or sedation.

For example, a first iso-electric suppression is illustrated on figure 6 and occurs at the iso-electric instant $t_{IES}$.

More specifically, the shorter the duration, the more sensitive is the person to a general anesthesia or sedation.

In the case where the EEG signal does not comprise any iso-electric suppression, one can only find a minimal bound on the duration between the reference instant and the iso-electric instant. It is still possible to estimate the third parameter and retrieve a relevant information for this person who is in this case appear with no sensitivity or a low sensitivity to general anesthesia or sedation.

Following this second option, one can determine a parameter relating to the sensitivity of the monitored person to general anesthesia or sedation, as soon as an iso-electric suppression takes place. This can occur less than 10 minutes following the loss of consciousness, and enables to obtain relevant information sooner than in the prior art.

As for the first parameter, a minimum quantity of anesthetic to inject so that the subject remains in general anesthesia or sedation may be determined based on the second or third parameter.

Usually, the iso-electric instant occurs after the alpha instant, but the iso-electric instant can also occur before the alpha instant.

It is thus a possibility to determine the third parameter before or after the first parameter.

To take into account this possibility, an adaptable version of the method is proposed, the adaptable version comprising both first and second options of the process described above. In the adaptable version, the method further comprises the determination of an induction instant following a predetermined induction duration after the reference instant.

The first minutes of anesthesia are currently known as an induction phase.

For instance, the predetermined induction duration can be set equal to 10 minutes, so that the induction instant, $t_{ind}$, occurs 10 minutes after the reference instant.

The method comprises also the determination of a final instant, $t_{fin}$, following a predetermined final duration after the induction instant.

For instance, the predetermined final duration can be set equal to 5 minutes, so that the final instant occurs 5 minutes after the induction instant.

The method enables to determine an alpha instant and an iso-electric instant. If the alpha instant is before the induction instant and the iso-electric instant, then the processor determines at the alpha instant the first parameter.

If the iso-electric instant is before the induction instant and the alpha instant, then the processor determines at the iso-electric instant the third parameter. In all cases, the processor determines a first estimate of the second parameter at the induction instant.

In all cases, the processor determines at the final instant, a second estimate of the second parameter, the first parameter if the alpha instant is before the final instant and the third parameter if the iso-electric instant is before the final instant.

It is possible that the alpha instant is after the induction instant or respectively the final instant, thus only a minimal bound on the duration between the reference instant and the alpha instant can be determined at the induction instant or respectively at the final instant.

Implementing the adaptable version of the method, one can determine as soon as possible a parameter relating to the sensitivity of the monitored person to general anesthesia or sedation: before the induction instant if a suppression of an $\alpha$-band or an iso-electric suppression occurs before the induction instant, or else at the induction instant. At the final instant, a second estimate of the second parameter, which is the rate of increase $\tan(\theta)$ or slope a is produced. As this second estimate is based on a longer duration of the EEG signal, the second estimate is more accurate and provides a more relevant information at the final instant.

Optionally to the different implementations of the method described so far, the reference instant may be determined following the steps of:

--fitting a curve of the frequency of maximal power as a function of time using a sigmoid function,
-- determining a range of values and a final value of the sigmoid function,
-- determining the reference instant as an instant when the sigmoid function reaches an intermediate value, a difference between the intermediate value and the final value being equal to 5% of the range of values.

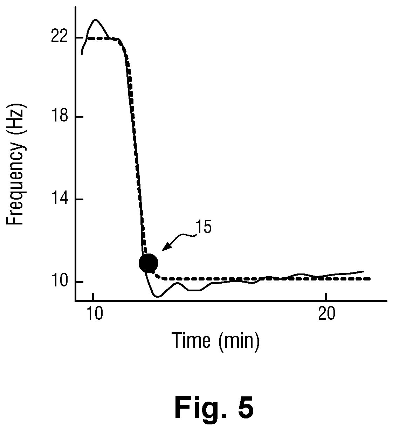

Figure 5 illustrates an example of the determination of a reference instant 15. In this example,

the fitted sigmoid follows the expression : $S(t) = \frac{12,26}{1 + exp(5,07 \times (t - 11,58))} + 10,38$ and

the reference instant is given by $\frac{1}{5,07} ln\left(\frac{1}{0.05} - 1\right) + 10,38 \approx 12,26 min$ .

This fitting allows to determine the portion of the spectrogram, where the beta band disappears and the alpha bands emerges, involving the [8-30] Hz frequencies. The result provides the exact location of the time continuous alpha band. This approach is more accurate than in the prior art and may be implemented even when the spectrogram is fragmented or the alpha band is not clearly visible.

*Predictive value of the estimated parameters*

The first parameter $(t_{\alpha S} - t_{ref})$, the second parameter ($a$) and the third parameter $(t_{IES} - t_{ref})$ can be used to predict the anesthesia sensitivity of the person and allow to estimate if the person belongs to :

- the 'IES group' of persons that experience an IES during a general anesthesia or a sedation (that is the persons who have an EEG signal comprising more than 12s of IES during the first 35 min of anesthesia or sedation), or
- the 'no IES group' of persons that do not experience an IES during a general anesthesia or a sedation (that is the persons who have an EEG signal comprising less than 12s of IES during the first 35 min of anesthesia or sedation).

To analyze the predictive value of the three parameters, they are first projected into the three normalized variables:

- a logarithm of the normalized alpha instant $t_{\alpha S} = log(t_{ind}/(t_{\alpha S} - t_{ref}) + 1)$ where $t_{ind}$=10 min.
- a normalized slope $a = a/\|a\|_\infty$, where $\|a\|_\infty$ is the maximum slope amplitude computed over the EEG recordings,
- a logarithm of the normalized iso-electric instant $t_{IES} = log(t_{ind}/(t_{IES} - t_{ref}) + 1)$ with $t_{ind}$=10 min.

One can deduce the parameters $a$ , $t_{\alpha S}$, $t_{IES}$, from the parameters $(t_{\alpha S} - t_{ref})$,($a$), $(t_{IES} - t_{ref})$ and vice-versa.

The parameters $(t_{\alpha S} - t_{ref})$,($a$), $(t_{IES} - t_{ref})$ or equivalently the parameters $a$ , $t_{\alpha S}$, $t_{IES}$ are further designated as the three parameters.

In a first study, 94 persons experience a general anesthesia and for each one of them the status IES / no IES was established on the one hand and the three parameters were estimated on the other hand. The groups IES / no IES appear well separated between the persons with lower values of $t_{\alpha S}$ and higher values of $t_{\alpha S}$, and equivalently with respect to the parameters $a$ and $t_{IES}$.

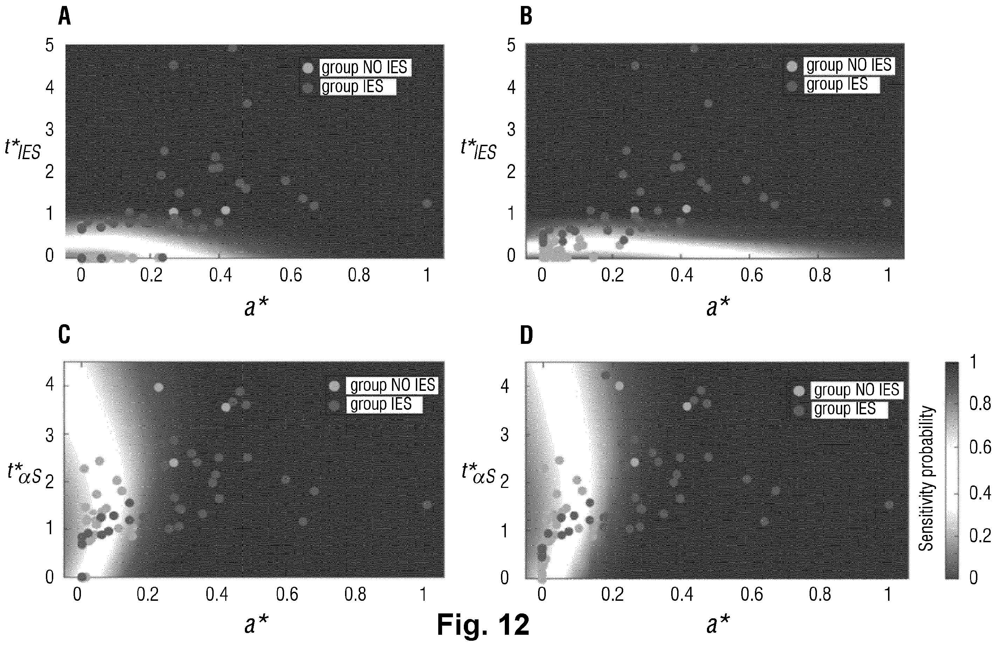

Figure 12 illustrates the separation of the groups with regards to the couple of parameters $a$ and $t_{IES}$, and the couple of parameters $a$ and $t_{\alpha S}$.

Parts A,B of figure 12 represents a diagram with the parameter $t_{IES}$.on the abscises axis and the parameter a on the ordinate axis.

Parts C,D of figure 12 represents a diagram with the parameter $t_{\alpha S}$.on the abscises axis and the parameter a on the ordinate axis.

Parts A,C of figure 12 relate to the induction phase, that is the first 15 minutes following the reference instant.

Parts B,D of figure 12 relate to the whole general anesthesia.

Each person of the 94 persons is represented on each diagram by a dot. Persons of the "IES group" correspond to a darker spot than the persons of the "No IES group".

In parts A,B of figure 12, persons of the "No IES group" are mostly located to the bottom left of the diagram , whereas persons of the "IES group" occupies the remaining space.

In parts C,D of figure 12, persons of the "No IES group" are mostly located to the left of the diagram , whereas persons of the "IES group" occupies the remaining space.

A probability map with different levels of grey is also represented on each diagram. The probability map is deduced from the logistic regression model between the two variables involved in the diagram.

To quantify the predictive value of the three parameters restricted to the induction phase a Monte-Carlo Cross Validation method was implemented. Such a method consists in creating multiple random splits of the patient data under the ratio of 70% training and 30% testing. The number of sampling was set to 50. In each sampling, several logistics models using various combinations of the three parameters are trained and tested. The combinations are compared by computing the mean ROC curves and AUC from these 50 experiments.

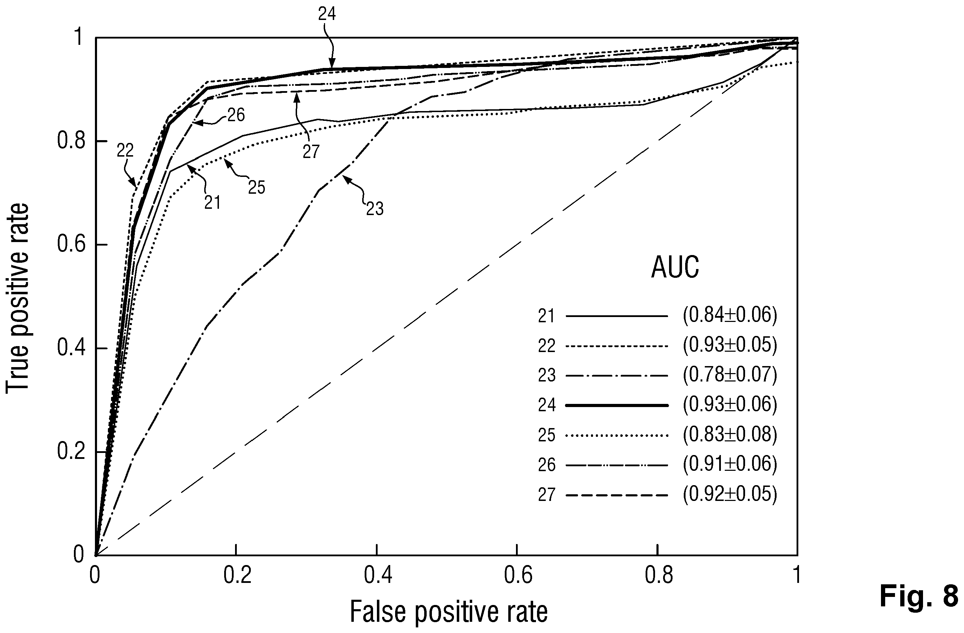

Figure 8 illustrates the result of this study, based on the 15 first minutes of the anesthesia

The curve 21 corresponds to a logistics model using only the parameter a and correspond to AUC = 0,84.

The curve 22 corresponds to a logistics model using only the parameter $t_{IES}$ and correspond to AUC = 0,93.

The curve 23 corresponds to a logistics model using only the parameter $t_{\alpha S}$ and correspond to AUC = 0,78.

The curve 24 corresponds to a logistics model using both parameters a , $t_{IES}$ and correspond to AUC = 0,93.

The curve 25 corresponds to a logistics model using both parameters a , $t_{\alpha S}$ and correspond to AUC = 0,83.

The curve 26 corresponds to a logistics model using both parameters $t_{\alpha S}$ ,$t_{IES}$ and correspond to AUC = 0,91.

The curve 27 corresponds to a logistics model using the three parameters a ,$t_{\alpha S}$, $t_{IES}$ and correspond to AUC = 0,92.

The couple (a , $t_{IES}$.) appears as the best predictor with an AUC=0.93±0.06, but this couple implies to wait sufficiently to collect the first IES. This result should be compared with the result obtained for the couple (a ,$t_{\alpha S}$) with lower predictive value AUC=0.83±0.08, but this couple can usually be estimated much earlier during the induction phase.

To further validate the predictions within the induction phase, a logistic regression was applied over the similar data, but base on the entire anesthesia time. Very similar ROC curves were obtained, showing that the sensitivity probability computed from the induction phase is robust and does not change during the entire anesthesia.

*Estimating a sensitivity of a person to general anesthesia or sedation*

The three parameters a , $t_{\alpha S}$, $t_{IES}$ characterize the sensitivity to general anesthesia or sedation and can be collected at different times during the induction phase.

The sensitivity may be expressed as the probability that the monitored person is part of the "IES group", probability written $P(IES = 1)$ or equivalently $P(S = 1)$.

It is possible to estimate a value of the sensitivity based on the alpha instant $t_{\alpha S}$, noted $P(IES = 1|t_{\alpha S})$ or equivalently $P(S = 1|t_{\alpha S})$ between the reference moment and the alpha moment, using logistic regression in the following form:

$$P\big(IES = 1\big|t_{\alpha S}\big) = \Big(1 + exp\big[-\big(\gamma + \delta_0(t_{\alpha S})\big)\big]\Big)^{-1}.$$

It is thus proposed a method for estimating a sensitivity of a subject to general anesthesia or sedation comprising :

- receiving an electroencephalogram EEG signal of a subject,
- processing the EEG signal comprising $\alpha$-bands for

-- defining for each instant of a plurality of instants, a part of the signal comprised in a time window comprising the instant, the part of the signal being related to the instant,
-- determining for each instant, a distribution of a power of the part of the signal as a function of frequency,
-- determining for each instant a maximum power frequency as a frequency of maximum power in the distribution, the maximum power frequency being related to the instant,
-- detecting a reference instant, so that the maximum power frequency as a function of time takes higher values before the reference instant than after,
-- detecting in a time frame following the reference instant, an alpha instant corresponding to a first occurrence of a suppression of an $\alpha$-band,

- estimating a first value of the sensitivity characterizing the risk of IES of the subject put under general anesthesia or sedation based on an alpha duration between the reference moment and the alpha moment.

The sensitivity involved here is the probability $P(IES = 1|t_{\alpha S})$. The following decision rule may be used in order to make medical decisions :

$P(IES = 1|t_{\alpha S}) \geq 0,5$ corresponds to the decision « the subject is sensitive to general anesthesia and is part of the group IES »

$P(IES = 1|t_{\alpha S}) < 0{,}5$ corresponds to the decision « the subject is not sensitive to general anesthesia and is part of the group No-IES »

This method may be further adapted to estimate other values of the sensitivity of the subject.

For instance, it is possible to estimate also a value of the sensitivity based on the alpha duration $t_{\alpha S}$ and the rate of increase or slope a,noted $P(IES = 1|t_{\alpha S}, a)$ using logistic regression in the following form:

$$P\big(IES = 1\big|t_{\alpha S}, a\big) = \Big(1 + exp\Big[-\big(\gamma + \delta_1(a) + \delta_2\big(t_{\alpha S}\big)\big)\Big]\Big)^{-1},$$

with $\gamma$ = -1.63, $\delta_1$ = 14.47, $\delta_2$ =-0.27.

It is possible to estimate a value of the sensitivity based on the iso-electric duration $t_{IES}$ and the rate of increase or slope $a$, $P(S = 1|t_{IES}, a)$ using logistic regression in the following form:

$$P\big(S = 1\big|t_{IES}, a\big) = \Big(1 + exp\Big[-\big(\gamma + \delta_1(a) + \delta_2\big(t_{IES}\big)\big)\Big]\Big)^{-1},$$

with y = -2.41, $\delta_1$ = 1.47 , $\delta_2$ =4.58.

Similar probabilities may be introduced such as $P(IES = 1|a)$ and $P(IES = 1|t_{IES})$. For all probabilities, the previous decision rule with the 0,5 threshold may be used in order to make medical decisions.

A decision tree procedure may also be implemented in order to quickly retrieve relevant information, make a decision on the status IES/No IES of the subject and define warnings at different instants or checkpoints about the sensitivity.

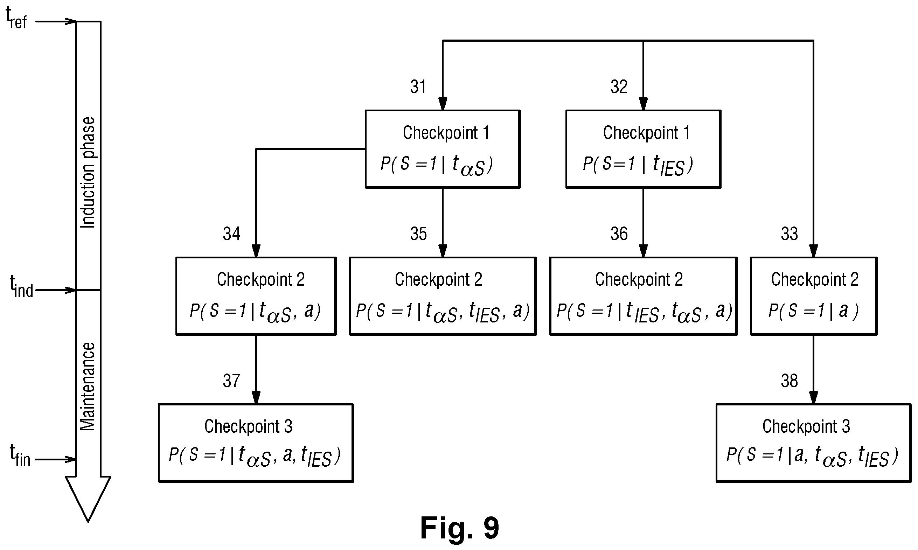

Figure 9 illustrates schematically the decision tree.

A temporal axis is represented on the left part of the figure 9, and the chronological sense is directed downwards of the figure 9. The reference instant $t_{ref}$ appears on the top of the axis, above the induction instant $t_{ind}$ and further down appears the final time $t_{fin}$.

The first checkpoint occurs after the reference instant $t_{ref}$, and consequently below $t_{ref}$ in figure 9.

The first checkpoint corresponds to :

- the first time an alpha suppression $\alpha S$ occurs, that is the alpha instant corresponding to the case referenced 31 in figure 9, and then an estimate of the sensitivity $P(S = 1|t_{\alpha S})$ can be produced based on the first parameter $t_{\alpha S}$ or equivalenly $t_{\alpha S}$;
- the first time an iso-electric suppression IES occurs, that is the iso-electric instant corresponding to the case referenced 32 in figure 9, and then an estimate of the sensitivity $P(S = 1|t_{IES})$ can be produced based on the third parameter $t_{IES}$ or equivalenly $t_{IES}$.

The second checkpoint is set at the induction instant $t_{ind}$, that is after an induction duration after the reference instant. The second checkpoint is temporally located about the end of the induction phase.

Possibly, no alpha suppression $\alpha S$ and no iso-electric suppression IES occurs before the second checkpoint corresponding to the case referenced 33 in figure 9. In this situation, the method does not comprise any first checkpoint. The slope can be estimated to the value zero, and a second estimate of the sensitivity $P(S = 1|a)$ can be produced at the second checkpoint.

If the first checkpoint corresponds to the alpha instant, corresponding to the case referenced 31 in figure 9, two possible cases can be separated at the second checkpoint.

In a first possible case referenced 34 in figure 9, no iso-electric suppression IES occurs before the second checkpoint. In this case, at the second checkpoint, an estimate of the sensitivity $P(S = 1|t_{\alpha S}, a)$ can be produced based on the first and second parameters $t_{\alpha S}$, a .

In the second possible case referenced 35 in figure 9, an iso-electric suppression IES occurs before the second checkpoint. In this case, at the second checkpoint, an estimate of the sensitivity $P(S = 1|t_{\alpha S}, t_{IES}, a)$ can be produced based on the three parameters.

If the first checkpoint corresponds to the iso-electric instant, corresponding to the case referenced 32 in figure 9, an alpha suppression $\alpha S$ occurs before the second checkpoint. In this case referenced 36 in figure 9, at the second checkpoint, an estimate of the sensitivity $P(S = 1|t_{\alpha S}, t_{IES}, a)$ can be produced based on the three parameters.

Finally, the third checkpoint is involved in the method if the three parameters have not been estimated at the second checkpoint, that is only in the cases referenced 33 and 34 in figure 9. For those cases, and if during the final duration between the induction instant and the final instant:

- an iso-electric suppression IES occurs in the case referenced 37 following the case 34, an estimate of the sensitivity $P(S = 1|t_{\alpha S}, t_{IES}, a)$ can be produced based on the three parameters at the third checkpoint,

- an alpha suppression $\alpha S$ and an iso-electric suppression IES occurs in the case referenced 38 following the case 33, an estimate of the sensitivity $P(S = 1|t_{\alpha S}, t_{IES}, a)$ can be produced based on the three parameters at the third checkpoint.

Following this decision tree, one can determine and confirm in real time the sensitivity of the monitored person to general anesthesia or sedation. After only 10 and 15 minutes following the loss of consciousness, one can obtain the estimates of the sensitivity.

*Updating a database for classification and parameters estimation*

Once a patient sensitivity is estimated following the decision tree presented above, the sensitivity is then established with certainty by determining the total time the brain has spent in iso-suppression IES at the end of the surgery. If the person has an EEG signal comprising less than 12s of IES during the first 35 min of anesthesia or sedation, then the person belongs to the 'no IES group'. The data of this new person is incorporated in a data basis, and after each monitoring of this type the data basis is completed and updated.

At each new person added in the data base, the settings of the logistic regression may be recomputed. This iterative procedure increases the precision of the prediction each time a person is incorporated in the data base.

In a second aspect of the present disclosure, a process of filtering an EEG signal is presented.

Different artifacts can be found in EEG signals.

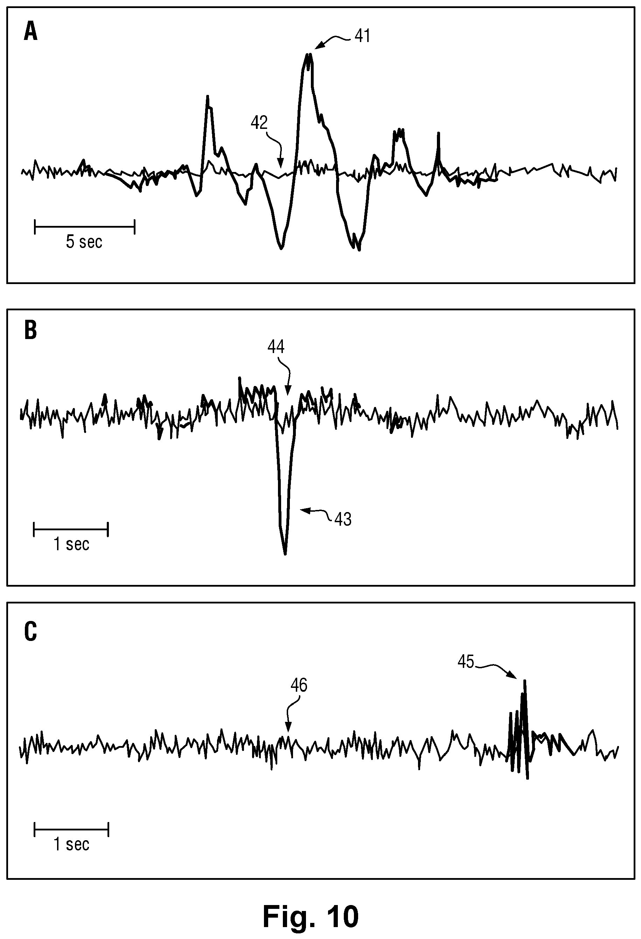

Figure 10 presents three examples of such artifacts: part A of figure 10 illustrates a motion artifact 41, part B of figure 10 illustrates an eye blink artifact 43, and part C of figure 10 illustrates a muscular artifact 45.

As an artifact appears in the EEG signal, no relevant processing of the signal can be carried out, preventing the anesthetist to retrieve any relevant information in the artifact section.

There is thus a need for a method to remove artifacts in real-time, so that they are replaced by a natural signal and that relevant statistics can be computed.

It is proposed a method to remove artifacts in electroencephalogram signal comprising the following steps :

- receiving an electroencephalogram signal of a subject,
- detecting an artifact signal within the signal,
- determining a ground signal using a portion of the signal immediately before or after the artifact,
- decomposing the artifact signal and respectively the ground signal using a discrete wavelet transform so as to determine, for each level of the transform an artifact projection of the artifact signal and respectively a ground projection of the ground signal, the artifact projection and respectively the ground projection being a projection of the artifact signal respectively the ground signal on a basis of the discrete wavelet transform,

- for each level m of the transform determining an artifact cumulative density function $F_m^{(art)}(x) = \frac{1}{N_m}\sum_{n=1}^{N_m} 1_{\left|w_{m,n}^{(art)}\right| < x}$ where $N_m$ is the total number of coefficients of the artifact projection at level m, $\left|w_{m,n}^{(art)}\right|$ is the absolute value of a n-th coefficient of the artifact projection at level m, and $1_{\left|w_{m,n}^{(art)}\right| < x}$ is an indicator function that returns the value 1 if $\left|w_{m,n}^{(art)}\right| < x$ and else the value 0,

- for each level m of the transform determining a ground cumulative density function $F_m^{(ref)}(x) = \frac{1}{N_m}\sum_{n=1}^{N_m} 1_{\left|w_{m,n}^{(ref)}\right| < x}$ where $N_m$ is the total number of coefficients of the artifact projection at level m, $\left|w_{m,n}^{(ref)}\right|$ is the absolute value of a n-th coefficient of the artifact projection at level m, and $1_{\left|w_{m,n}^{(ref)}\right| < x}$ is an indicator function that returns the value 1 if $\left|w_{m,n}^{(ref)}\right| < x$ and else the value 0,

- for each level m and for each artifact coefficient $w_{m,n}^{(art)}$ determining a corrected coefficient $w_{m,n}^{(corr)}$ of a corrected projection, the artifact coefficient and the corrected coefficient being similarly indexed, following the sub-steps:

-- determining the value $F_m^{(art)}\left(\left|w_{m,n}^{(art)}\right|\right)$ ,

-- determining an intermediate value $x$ verifying $F_m^{(ref)}(x) = F_m^{(art)}\left(\left|w_{m,n}^{(art)}\right|\right)$ ,

-- setting the corrected coefficient $w_{m,n}^{(corr)}$ equal to $w_{m,n}^{(art)} \times min\left\{1, \frac{x}{\left|w_{m,n}^{(art)}\right|}\right\}$ ,

- determining a corrected signal using the discrete wavelet transform based on the corrected projection,
- replacing in the signal the artifact signal by the corrected signal.

The novel thresholding procedure is an adaptive method using quantile normalization of the wavelet coefficients to attenuate coefficients of the artifact signal.

The use of the cumulative density function enables, for each level of the transform, to compare globally coefficients of the artifact signal and coefficients of the ground signal and to modify the coefficients of the artifact signal so as to set the power of the artifact projection inferior or equal to the power of the ground projection.

Since the distribution of energy across frequency bands for physiological EEG signal should be continuous, no abrupt changes in the power of a band are expected. An artifact is precisely such an abrupt change. Setting the power of the artifact projection equal to the power of the ground projection and defining a corrected signal based on this correction enables to remove the artifacts, replace it by a natural signal so that relevant statistics can be computed.

Also, this method can be implemented in real-time.

The method applies to any EEG signal. In particular, the method may be applied simultaneously with the computer-implemented method for assisting a general anesthesia or a sedation of a subject as described previously.

The present disclosure also concerns a processor configured to implement a method to remove artifacts in electroencephalogram signal as described above.

The general anesthesia or sedation assisting device as described previously comprises a processor that can be also configured to implement the method to remove artifacts in electroencephalogram signal as described above.

The method comprises a first step of receiving an electroencephalogram signal of a subject, this signal can be denoted as the function $x(t)$ of time $t$.

In a second step, an artifact signal is detected within the signal. This detection can be based on a detection of an abnormal high amplitude signal or by thresholding the signal power to detect non-physiological levels. An artifact time window is thus defined to comprise the detected artifact.

The EEG signal limited to the artifact time window defines an artifact signal $x^{(art)}(t)$.

In a third step, a ground signal is determined using a portion of the signal immediately before or after the artifact. This determination may be implemented by selecting within the EEG signal a ground time window located before the artifact time window and/or after the artifact time window. The ground time window can have the same duration as the artifact window.

The EEG signal limited to the ground time window defines a ground signal $x^{(ref)}(t)$.

In a fourth step, both the artifact signal and the ground signal are decomposed using a discrete wavelet transform so as to determine, for each level of the transform an artifact projection of the artifact signal and a ground projection of the ground signal.

The artifact projection is a projection of the artifact signal on a basis of the discrete wavelet transform.

The ground projection is a projection of the ground signal on a basis of the discrete wavelet transform.

The basis of the discrete wavelet transform consists in :

- a first family of functions $\psi_{m,n}(t)$ defined via a first reference function $\psi(t)$ or wavelet function $\psi(t)$, with the relation $\psi_{m,n}(t) = 2^{-m/2}\psi(2^{-m}t - n)$,,

- a second family of functions $\phi_{m,n}(t)$ defined via a second reference function $\phi(t)$ or scaling function $\phi(t)$, with the relation $\phi_{m,n}(t) = 2^{-m/2}\phi(2^{-m}t - n)$.

A wavelet transform to decompose at level M a signal $x(t)$ into time-frequency components leads to the following expression:

$$x(t) = \sum_n c_{M,n}\phi_{M,n}(t) + \sum_{m=1}^{M} \sum_n d_{m,n}\psi_{m,n}(t)$$

where m is an integer taking values from 1 to M, n is an integer taking a number $N_m$ values depending on m, $c_{m,n} = \langle x, \phi_{m,n}\rangle$ is the coefficient of projection of x(t) on function $\phi_{m,n}(t)$ and $d_{m,n} = \langle x, \psi_{m,n}\rangle$ the coefficient of projection of x(t) on function $\psi_{m,n}(t)$.

The scalar product is defined by $\langle f, g\rangle = \Sigma_{t\in Z} f(t)g(t)$.

The signal $x(t)$ is decomposed onto an orthogonal basis obtained by dilating and translating in time the wavelet function $\psi(t)$ and scaling function $\phi(t)$.

The decomposition of the artifact signal $x^{(art)}(t)$ produces the artifact coefficients $d_m^{(art)}, c_M^{(art)}$

The decomposition of the ground signal $x^{(ref)}(t)$ produces the ground coefficients $d_m^{(ref)}, c_M^{(ref)}$.

To simplify the description, all coefficients can be renamed in the following manner:

$w_{m,n} = d_{m,n}$ for $m = 1, ... , M$ and $w_{M+1,n} = c_{M,n}$ defining : $w_{m,n}^{(art)}, w_{m,n}^{(ref)}$

$w_{m,n}$ is a notation to designate the coefficients indexed by the integer n : $d_{m,n}$ or $c_{M,n}$.

In a fifth step, an artifact cumulative density function $F_m^{(art)}(x)$ is determined for each level m of the transform with the following definition $F_m^{(art)}(x) = \frac{1}{N_m}\sum_{n=1}^{N_m} 1_{\left|w_{m,n}^{(art)}\right|<x}$ where $N_m$ is the total number of coefficients of the artifact projection at level m, $\left|w_{m,n}^{(art)}\right|$ is the absolute value of a n-th coefficient of the artifact projection at level m, and $1_{\left|w_{m,n}^{(art)}\right|<x}$ is an indicator function that returns the value 1 if $\left|w_{m,n}^{(art)}\right| < x$ and else the value 0.

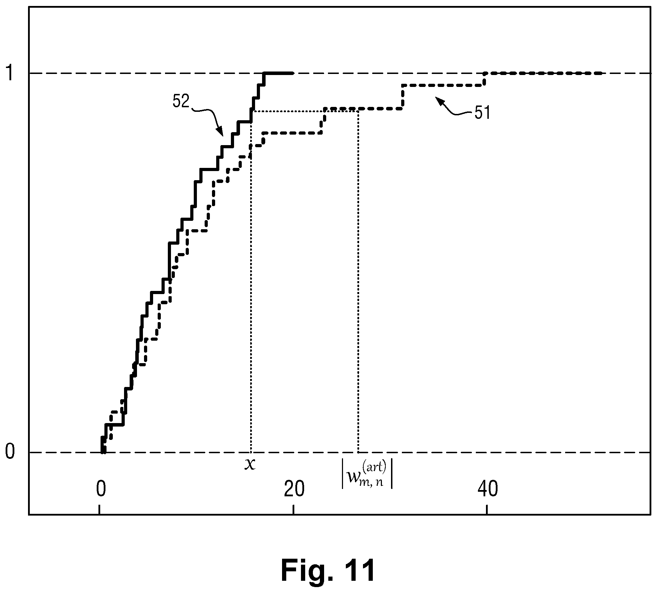

Figure 11 represents schematically a cumulative density function $F_m^{(art)}(x)$ 51 as a function of x. The cumulative density function is a weakly monotonically increasing function taking values between 0 and 1. The cumulative density function takes the value 0 for x=0, and eventually reaches the value 1 for sufficiently high values of x.

In a sixth step, a ground cumulative density function is determined for each level $m$ of the transform with the following definition $F_m^{(ref)}(x) = \frac{1}{N_m}\sum_{n=1}^{N_m} 1_{\left|w_{m,n}^{(ref)}\right|<x}$ where $N_m$ is the total number of coefficients of the artifact projection at level $m$, $\left|w_{m,n}^{(ref)}\right|$ is the absolute value of a n-th coefficient of the artifact projection at level $m$, and $1_{\left|w_{m,n}^{(ref)}\right|<x}$ is an indicator function that returns the value 1 if $\left|w_{m,n}^{(ref)}\right| < x$ and else the value 0.

Figure 11 represents schematically a cumulative density function $F_m^{(ref)}(x)$ 52 as a function of x.

In a seventh step, for each level m and for each artifact coefficient $w_{m,n}^{(art)}$ a corrected coefficient $w_{m,n}^{(corr)}$ of a corrected projection is determined, the artifact coefficient and the corrected coefficient being similarly indexed. This determination follows the sub-steps:

-- determining the value $F_m^{(art)}\left(\left|w_{m,n}^{(art)}\right|\right)$ - Figure 11 represents schematically this value ;

-- determining an intermediate value x verifying $F_m^{(ref)}(x) = F_m^{(art)}\left(\left|w_{m,n}^{(art)}\right|\right)$ - Figure 11 represents schematically the intermediate value x ;

- setting the corrected coefficient $w_{m,n}^{(corr)}$ equal to $w_{m,n}^{(art)} \times min\left\{1, \dfrac{x}{\left|w_{m,n}^{(art)}\right|}\right\}$,

Using this setting, the artifact coefficient $w_{m,n}^{(art)}$ is globally compared to the entire distribution of ground coefficients.

In an eighth step, a corrected signal $x^{(corr)}(t)$ is determined using the discrete wavelet transform based on the corrected projection. More precisely, the seventh step enables to find a set of corrected coefficients $w_{m,n}^{(corr)}$ in correspondence with the set of artifact coefficients $w_{m,n}^{(art)}$. $d_{m,n}^{(corr)}$ and $c_{m,n}^{(corr)}$ can thus be defined with the previsously mentioned relations : $w_{m,n} = d_{m,n}$ for $m = 1, ... , M$ and $w_{M+1,n} = c_{M,n}$.

The corrected signal $x^{(corr)}(t)$ is then determined through the expression

$$x^{(corr)}(t) = \sum_n c_{M,n}^{(corr)} \phi_{M,n}(t) + \sum_{m=1}^{M} \sum_n d_{m,n}^{(corr)} \psi_{m,n}(t)$$

Finally, in a ninth step, the artifact signal $x^{(art)}(t)$ is replaced in the signal EEG by the corrected signal $x^{(corr)}(t)$.

The artifact is thus removed from the EEG signal and replaced by a corrected signal which power is inferior or equal to the power of the ground signal neighboring the corrected signal.

Using such a method, the artifacts in figure 10 have been removed.

Part A of figure 10 illustrates a signal 42 obtained from the EEG that initially comprises the motion artifact 41 and to which the method was applied.

In the same way, part B of figure 10 illustrates a signal 44 obtained from the EEG that initially comprises the eye blink artifact 43 and to which the method was applied and part C of figure 10 illustrates a signal 46 obtained from the EEG that initially comprises a muscular artifact 45 and to which the method was applied. This method can be implemented in real-time. As the EEG signal is acquired, a corrected EEG signal can be progressively produced using the method. Advantageously, the presented method enables a reliable monitoring of the EEG signal. When the EEG signal carries an artifact, a spectrogram produced from this signal shows also artifacts. Such noisy perturbations can affect the calculation of spectrogram and the estimates of the depth of anesthesia. The corrected EEG signal may be used to produce a spectrogram with which the parameters relating to the sensitivity of a subject to general anesthesia or sedation can be computed.

**Claims**

1. Computer-implemented method for assisting a general anesthesia or a sedation of a subject (2) initially conscious that is put into an artificial coma, comprising the steps of:

   - receiving an electroencephalogram EEG signal of the subject, the EEG signal comprising alpha bands,
   - processing the received EEG signal, the processing comprising:

     -- defining a sliding time window,
     -- determining a truncated signal, the truncated signal being equal to the part of the EEG signal within the sliding window and equal to zero outside the sliding time window,
     -- determining a distribution of a power of the truncated signal as a function of frequency,
     -- determining a maximum power, fmax, from the distribution of the power of the truncated signal,
     -- extracting the frequency of maximum power of the received EEG signal as a function of time, fmax(t), as the sliding time window slides along the received EEG signal,
     -- determining a reference time instant, tref, by curve fitting fmax(t) using a curve fitting function, tref being the time instant when the curve fitting function reaches an intermediate value such that the difference between the intermediate value and a minimum value of the curve fitting function is equal to 5% of a range of values of the curve fitting function, so that the maximum power frequency as a function of time fmax(t) takes higher values before the reference time instant than after, and

-- detecting an alpha time instant, tαs, in the received EEG signal, the alpha time instant corresponding to a first occurrence of a suppression of an α-band, the alpha time instant being in a time frame posterior to the reference time instant tref,

- estimating a first parameter as the duration between the reference time instant tref, and the alpha time instant tαs, said first parameter defining a sensitivity of the subject to a general anesthesia or sedation, and
- using the first parameter to assist the general anesthesia or the sedation of the subject.

2. Method according to claim 1, further comprising:

- determining time spans in the truncated signal, a suppression of an α-band extending on each time span,
- determining a proportion of the time spans in the sliding time window, as a function of time, as the sliding time window slides along the received EEG signal,
- estimating a second parameter as a rate of increase of the proportion of the time spans in the signal, said second parameter relating to a sensitivity of the subject to a general anesthesia or sedation.

3. Method according to claim 2, further comprising:

- detecting in a time frame posterior to the reference time instant tref, an iso-electric time instant, $t_{IES}$, of a first occurrence of an iso-electric suppression,
- estimating a third parameter as the duration between the reference time instant tref, and the iso-electric time instant $t_{IES}$, said third parameter relating to a sensitivity of the subject to a general anesthesia or sedation.

4. Method according to claim 3, comprising:

- determining an induction time instant, tind, posterior to the reference time instant tref, the induction time instant tind and the reference time instant tref being separated by a predetermined induction duration,
- determining a final time instant, tfin, posterior to the induction time instant tind, the final time instant tfin and the induction time instant tind being separated by a predetermined final duration,
- if the alpha time instant tαs, is before the induction time instant tind and the iso-electric time instant $t_{IES}$, determining at the alpha time instant the first parameter,
- if the iso-electric time instant $t_{IES}$ is before the induction time instant tind and the alpha time instant, determining at the iso-electric time instant $t_{IES}$ the third parameter,
- determining a first estimate of the second parameter at the induction time instant tind,
- determining at the final time instant tfin, a second estimate of the second parameter, the first parameter if the alpha time instant tαs is before the final time instant tfin and the third parameter if the iso-electric time instant $t_{IES}$ is before the final time instant tfin.

5. Method according to any of the preceding claims wherein :

- detecting the reference time instant tref comprises

--fitting fmax(t), the frequency of maximum power of the received EEG signal as a function of time using a sigmoid function,
-- determining a range of values and a final value of the sigmoid function,
-- determining the reference time instant as an instant when the sigmoid function reaches the intermediate value so that a difference between the intermediate value and the final value being equal to 5% of the range of values.

6. Method according to any of the preceding claims further comprising the following steps :

- detecting an artifact signal within the received EEG signal,
- determining a ground signal using a portion of the received EEG signal immediately before or after the artifact,
- decomposing the artifact signal and respectively the ground signal using a discrete wavelet transform so as to determine, for each level of the transform an artifact projection of the artifact signal and respectively a ground projection of the ground signal, the artifact projection and respectively the ground projection being a projection of the artifact signal respectively the ground signal on a basis of the discrete wavelet transform,
- for each level m of the transform determining an artifact cumulative density function

$$F_m^{(art)}(x) = \frac{1}{N_m}\sum_{n=1}^{N_m} 1_{\left|w_{m,n}^{(art)}\right|<x}$$ where $N_m$ is the total number of coefficients of the artifact projection at level m, $\left|w_{m,n}^{(art)}\right|$ is the absolute value of a n-th coefficient of the artifact projection at level m, and $1_{\left|w_{m,n}^{(art)}\right|<x}$ is an indicator function that returns the value 1 if $\left|w_{m,n}^{(art)}\right| < x$ and else the value 0,

- for each level m of the transform determining a ground cumulative density function $$F_m^{(ref)}(x) = \frac{1}{N_m}\sum_{n=1}^{N_m} 1_{\left|w_{m,n}^{(ref)}\right|<x}$$ where $N_m$ is the total number of coefficients of the artifact projection at level m, $\left|w_{m,n}^{(ref)}\right|$ is the absolute value of a n-th coefficient of the artifact projection at level m, and $1_{\left|w_{m,n}^{(ref)}\right|<x}$ is an indicator function that returns the value 1 if $\left|w_{m,n}^{(ref)}\right| < x$ and else the value 0,

- for each level m and for each artifact coefficient $w_{m,n}^{(art)}$ determining a corrected coefficient $w_{m,n}^{(corr)}$ of a corrected projection, the artifact coefficient and the corrected coefficient being similarly indexed, following the sub-steps:

-- determining the value $F_m^{(art)}\left(\left|w_{m,n}^{(art)}\right|\right)$,

-- determining an intermediate value x verifying $F_m^{(ref)}(x\ ) = F_m^{(art)}\left(\left|w_{m,n}^{(art)}\right|\right)$ ,

- setting the corrected coefficient $w_{m,n}^{(corr)}$ equal to $w_{m,n}^{(art)} \times min\left\{1, \frac{x}{\left|w_{m,n}^{(art)}\right|}\right\}$,

- determining a corrected signal using the discrete wavelet transform based on the corrected projection,
- replacing in the received EEG signal the artifact signal by the corrected signal.

**7.** Method according to any of the claims 1 to 6 comprising a determination of a minimum quantity of anesthetic to inject so that the subject (2) remains in general anesthesia or sedation, the determination being based on the first parameter.

**8.** General anesthesia or sedation assisting device (1) comprising a processor (3) configured for implementing the computer implemented method as claimed in claim 1 to 7.

**9.** A computer-readable medium comprising computer-executable instructions which, when executed by a computer, cause the computer to carry out the method according to any of claims 1 to 7.

**Patentansprüche**

**1.** Computerimplementiertes Verfahren zum Unterstützen einer Vollnarkose oder einer Sedierung eines Subjekts (2), dem eine Dosis eines Narkosemittels zum Einleiten einer Vollnarkose oder einer Sedierung verabreicht wurde, umfassend die Schritte:

- Empfangen eines Elektroenzephalogrammsignals, EEG-Signal, des Subjekts, das EEG-Signal umfassend Alpha-Bänder,
- Verarbeiten des empfangenen EEG-Signals, das Verarbeiten umfassend

-- Definieren eines gleitenden Zeitfensters,
-- Bestimmen eines gekürzten Signals, wobei das gekürzte Signal innerhalb des gleitenden Fensters gleich dem Teil des EEG-Signals und außerhalb des gleitenden Zeitfensters gleich null ist,
-- Bestimmen einer Verteilung einer Leistung des gekürzten Signals als eine Funktion einer Frequenz,

-- Bestimmen einer maximalen Leistung, fmax, aus der Verteilung der Leistung des gekürzten Signals,

-- Extrahieren der Frequenz der maximalen Leistung des empfangenen EEG-Signals als eine Funktion von Zeit, fmax(t), während das gleitende Zeitfenster entlang des empfangenen EEG-Signals gleitet,

-- Bestimmen eines Referenzzeitpunkts, tref, durch Kurvenanpassung von fmax(t) unter Verwendung einer Kurvenanpassungsfunktion, wobei tref der Zeitpunkt ist, an dem die Kurvenanpassungsfunktion einen Zwischenwert derart erreicht, dass die Differenz zwischen dem Zwischenwert und einem minimalen Wert der Kurvenanpassungsfunktion gleich 5 % eines Wertebereichs der Kurvenanpassungsfunktion ist, sodass die Frequenz der maximalen Leistung als eine Funktion der Zeit fmax(t) vor dem Referenzzeitpunkt höhere Werte als danach annimmt, und

-- Erfassen eines Alpha-Zeitpunkts, $t_{\alpha S}$, in dem empfangenen EEG-Signal, wobei der Alpha-Zeitpunkt einem ersten Auftreten einer Unterdrückung eines $\alpha$-Bands entspricht, wobei der Alpha-Zeitpunkt in einem Zeitrahmen nach dem Referenzzeitpunkt tref liegt,

- Schätzen eines ersten Parameters als die Dauer zwischen dem Referenzzeitpunkt tref und dem Alpha-Zeitpunkt $t_{\alpha S}$, wobei der erste Parameter eine Empfindlichkeit des Subjekts gegenüber einer Vollnarkose oder Sedierung definiert, und

- Verwenden des ersten Parameters, um die Vollnarkose oder die Sedierung des Subjekts zu unterstützen.

2. Verfahren nach Anspruch 1, ferner umfassend:

- Erfassen, in dem gleitenden Fenster, für jeden Zeitpunkt, der Zeitspanne, die durch die Unterdrückung des $\alpha$-Bands eingenommen wird,

- Schätzen eines zweiten Parameters, der eine Anstiegsrate des Anteils des empfangenen EEG-Signals ist, der durch die Zeitspannen in dem empfangenen EEG-Signal eingenommen wird, wobei sich der zweite Parameter auf eine Empfindlichkeit des Subjekts gegenüber einer Vollnarkose oder Sedierung bezieht.

3. Verfahren nach Anspruch 2, ferner umfassend:

- Erfassen, in einem Zeitrahmen nach dem Referenzzeitpunkt tref, eines isoelektrischen Zeitpunkts, tIES, eines ersten Auftretens einer isoelektrischen Unterdrückung,

- Schätzen eines dritten Parameters als die Dauer zwischen dem Referenzzeitpunkt tref und dem isoelektrischen Zeitpunkt tIES, wobei sich der dritte Parameter auf eine Empfindlichkeit des Subjekts gegenüber einer Vollnarkose oder Sedierung bezieht.

4. Verfahren nach Anspruch 3, umfassend:

- Bestimmen eines Induktionszeitpunkts, tind, nach dem Referenzzeitpunkt tref, wobei der Induktionszeitpunkt tind und der Referenzzeitpunkt tref durch eine zuvor bestimmte Induktionsdauer getrennt sind,

- Bestimmen eines Endzeitpunkts, tend, nach dem Induktionszeitpunkt tind, wobei der Endzeitpunkt tend und der Induktionszeitpunkt tind durch eine zuvor bestimmte Enddauer getrennt sind,

- falls der Alpha-Zeitpunkt $t_{\alpha S}$ vor dem Induktionszeitpunkt tind und dem isoelektrischen Zeitpunkt tIES liegt, Bestimmen, zu dem Alpha-Zeitpunkt, des ersten Parameters,

- falls der isoelektrische Zeitpunkt tIES vor dem Induktionszeitpunkt tind und dem Alpha-Zeitpunkt liegt, Bestimmen, zu dem isoelektrischen Zeitpunkt tIES, des dritten Parameters,

- Bestimmen einer ersten Schätzung des zweiten Parameters zu dem Induktionszeitpunkt tind,

- Bestimmen, zu dem Endzeitpunkt tend, einer zweiten Schätzung des zweiten Parameters, des ersten Parameters, falls der Alpha-Zeitpunkt $t_{\alpha S}$ vor dem Endzeitpunkt tend liegt, und des dritten Parameters, falls der isoelektrische Zeitpunkt tIES vor dem Endzeitpunkt tend liegt.

5. Verfahren nach einem der vorstehenden Ansprüche, wobei:

- das Erfassen des Referenzzeitpunkts tref umfasst

-- Anpassen von fmax(t), der Frequenz der maximalen Leistung des empfangenen EEG-Signals als eine Funktion der Zeit unter Verwendung einer Sigmoidfunktion,

-- Bestimmen eines Wertebereichs und eines Endwerts der Sigmoidfunktion,

-- Bestimmen des Referenzzeitpunkts als einen Punkt, an dem die Sigmoidfunktion den Zwischenwert erreicht, sodass eine Differenz zwischen dem Zwischenwert und dem Endwert gleich 5 % des Wertebereichs ist.

6. Verfahren nach einem der vorstehenden Ansprüche, ferner umfassend die folgenden Schritte:

- Erfassen eines Artefaktsignals innerhalb des empfangenen EEG-Signals,
- Bestimmen eines Massesignals unter Verwendung eines Teils des empfangenen EEG-Signals unmittelbar vor oder nach dem Artefakt,
- Zerlegen des Artefaktsignals beziehungsweise des Massesignals unter Verwendung einer diskreten Wavelet-Transformation, um für jede Ebene der Transformation eine Artefaktprojektion des Artefaktsignals beziehungsweise eine Masseprojektion des Massesignals zu bestimmen, wobei die Artefaktprojektion beziehungsweise die Masseprojektion eine Projektion des Artefaktsignals beziehungsweise des Massesignals auf einer Basis der diskreten Wavelet-Transformation ist,
- für jede Ebene m der Transformation, Bestimmen einer kumulativen Artefaktdichtefunktion

$$F_m^{(art)}(x) = \frac{1}{N_m} \sum_{n=1}^{N_m} 1_{\left|w_{m,n}^{(art)}\right| < x}$$ , wobei $N_m$ die Gesamtzahl von Koeffizienten der Artefaktprojektion auf Ebene m ist, $\left|w_{m,n}^{(art)}\right|$ der Absolutwert eines n-ten Koeffizienten der Artefaktprojektion auf Ebene m ist und

$1_{\left|w_{m,n}^{(art)}\right| < x}$ eine Indikatorfunktion ist, die den Wert 1 falls $\left|w_{m,n}^{(art)}\right| < x$ und sonst den Wert 0 zurückgibt,

- für jede Ebene m der Transformation, Bestimmen einer kumulativen Massedichtefunktion

$$F_m^{(ref)}(x) = \frac{1}{N_m} \sum_{n=1}^{N_m} 1_{\left|w_{m,n}^{(ref)}\right| < x}$$ , wobei $N_m$ die Gesamtzahl von Koeffizienten der Artefaktprojektion auf Ebene m ist, $\left|w_{m,n}^{(ref)}\right|$ der Absolutwert eines n-ten Koeffizienten der Artefaktprojektion auf Ebene m ist und $1_{\left|w_{m,n}^{(ref)}\right| < x}$ eine Indikatorfunktion ist, die den Wert 1 falls $\left|w_{m,n}^{(art)}\right| < x$ und sonst den Wert 0 zurückgibt,

- für jede Ebene m und für jeden Artefaktkoeffizienten $w_{m,n}^{(art)}$, Bestimmen eines korrigierten Koeffizienten

$w_{m,n}^{(korr)}$ einer korrigierten Projektion, wobei der Artefaktkoeffizient und der korrigierte Koeffizient ähnlich indiziert werden, nach den folgenden Unterschritten:

-- Bestimmen des Werts $F_m^{(art)}\left(\left|w_{m,n}^{(art)}\right|\right)$,
-- Bestimmen eines Zwischenwerts x zum Überprüfen von

$$F_m^{(ref)}(x) = F_m^{(art)}\left(\left|w_{m,n}^{(art)}\right|\right),$$

- Einstellen des korrigierten Koeffizienten $w_{m,n}^{(korr)}$ gleich $w_{m,n}^{(art)} \times \min\left\{1, \frac{x}{\left|w_{m,n}^{(art)}\right|}\right\}$,

- Bestimmen eines korrigierten Signals unter Verwendung der diskreten Wavelet-Transformation basierend auf der korrigierten Projektion,
- Ersetzen, in dem empfangenen EEG-Signal, des Artefaktsignals durch das korrigierte Signal.

7. Verfahren nach einem der Ansprüche 1 bis 6, umfassend eine Bestimmung einer Mindestmenge an zu injizierendem Narkosemittel, sodass das Subjekt (2) in Vollnarkose oder Sedierung bleibt, wobei die Bestimmung auf dem ersten Parameter basiert.

**8.** Unterstützungsvorrichtung (1) für die Vollnarkose oder Sedierung, umfassend einen Prozessor (3), der zum Implementieren des computerimplementierten Verfahrens nach Anspruch 1 bis 7 konfiguriert ist.

**9.** Computerlesbares Medium, umfassend computerausführbare Anweisungen, die, wenn sie durch einen Computer ausgeführt werden, den Computer veranlassen, das Verfahren nach einem der Ansprüche 1 bis 7 durchzuführen.

**Revendications**

**1.** Procédé mis en œuvre par ordinateur permettant d'assister une anesthésie générale ou une sédation d'un sujet (2) qui a reçu une dose d'anesthésique pour induire une anesthésie générale ou une sédation, comprenant les étapes consistant à :

- recevoir un signal EEG d'électroencéphalogramme du sujet, le signal EEG comprenant des bandes alpha,
- traiter le signal EEG reçu, le traitement comprenant

-- l'établissement d'une fenêtre temporelle glissante,
-- la détermination d'un signal tronqué, le signal tronqué étant égal à la partie du signal EEG à l'intérieur de la fenêtre coulissante et égal à zéro à l'extérieur de la fenêtre de temps coulissante,
-- la détermination d'une distribution d'une puissance du signal tronqué en fonction de la fréquence,
-- la détermination d'une puissance maximale, fmax, à partir de la distribution de la puissance du signal tronqué,
-- l'extraction de la fréquence de puissance maximale du signal EEG reçu en fonction du temps, fmax(t), au fur et à mesure que la fenêtre temporelle glisse le long du signal EEG reçu,
-- la détermination d'un instant de temps de référence, tref, en ajustant la courbe fmax(t) à l'aide d'une fonction d'ajustement de courbe, tref étant l'instant où la fonction d'ajustement de courbe atteint une valeur intermédiaire telle que la différence entre la valeur intermédiaire et une valeur minimale de la fonction d'ajustement de courbe est égale à 5 % d'une plage de valeurs de la fonction d'ajustement de courbe, de sorte que la fréquence de puissance maximale en fonction du temps fmax(t) prend des valeurs plus élevées avant l'instant de temps de référence qu'après, et
-- la détection d'un instant alpha, $t_{\alpha S}$, dans le signal EEG reçu, l'instant alpha correspondant à une première occurrence d'une suppression d'une bande $\alpha$, l'instant alpha se situant dans un cadre temporel postérieur à l'instant de temps de référence tref,

- estimer un premier paramètre en tant que durée entre l'instant de temps de référence tref et l'instant alpha $t_{\alpha S}$, ledit premier paramètre définissant une sensibilité du sujet à une anesthésie générale ou à une sédation, et
- utiliser le premier paramètre pour assister l'anesthésie générale ou la sédation du sujet.

**2.** Procédé selon la revendication 1, comprenant en outre :

- la détection dans la fenêtre coulissante, pour chaque instant de temps, de l'intervalle de temps occupé par la suppression de la bande $\alpha$,
- l'estimation d'un second paramètre qui est un taux d'augmentation de la proportion du signal EEG reçu occupée par les intervalles de temps dans le signal EEG reçu, ledit second paramètre étant lié à la sensibilité du sujet à une anesthésie générale ou à une sédation.

**3.** Procédé selon la revendication 2, comprenant en outre :

- la détection, dans un laps de temps postérieur à l'instant de temps de référence tref, d'un instant iso-électrique, tIES, d'une première occurrence d'une suppression iso-électrique,
- l'estimation d'un troisième paramètre comme la durée entre l'instant de temps de référence tref et l'instant iso-électrique tIES, ce troisième paramètre étant relatif à la sensibilité du sujet à une anesthésie générale ou à une sédation.

**4.** Procédé selon la revendication 3, comprenant :

- la détermination d'un instant d'induction, tind, postérieur à l'instant de temps de référence tref, l'instant d'induction tind et l'instant de temps de référence tref étant séparés par une durée d'induction prédéterminée,

- la détermination d'un instant final, tfin, postérieur à l'instant d'induction tind, l'instant final tfin et l'instant d'induction tind étant séparés par une durée finale prédéterminée,

- si l'instant alpha $t_{\alpha S}$ est antérieur à l'instant d'induction tind et à l'instant iso-électrique tIES, la détermination à l'instant alpha du premier paramètre,

- si l'instant iso-électrique tIES est antérieur à l'instant d'induction tind et à l'instant alpha, la détermination à l'instant iso-électrique tIES du troisième paramètre,

- la détermination d'une première estimation du second paramètre à l'instant d'induction tind,

- la détermination, à l'instant final tfin, d'une seconde estimation du second paramètre, du premier paramètre si l'instant alpha $t_{\alpha S}$ est antérieur à l'instant final tfin et du troisième paramètre si l'instant iso-électrique tIES est antérieur à l'instant final tfin.

5. Procédé selon l'une quelconque des revendications précédentes, dans lequel :

- la détection de l'instant de temps de référence tref comprend

-- l'ajustement de fmax(t), la fréquence de la puissance maximale du signal EEG reçu en fonction du temps à l'aide d'une fonction sigmoïde,
-- la détermination d'une plage de valeurs et d'une valeur finale de la fonction sigmoïde,
-- la détermination de l'instant de temps de référence comme un instant où la fonction sigmoïde atteint la valeur intermédiaire de telle sorte que la différence entre la valeur intermédiaire et la valeur finale soit égale à 5 % de la plage de valeurs.

6. Procédé selon l'une quelconque des revendications précédentes, comprenant en outre les étapes suivantes :

- la détection d'un signal d'artefact dans le signal EEG reçu,
- la détermination d'un signal de masse à l'aide d'une partie du signal EEG reçu immédiatement avant ou après l'artefact,
- la décomposition du signal d'artefact et respectivement le signal de fond à l'aide d'une transformée en ondelettes discrète afin de déterminer, pour chaque niveau de la transformée, une projection d'artefact du signal d'artefact et respectivement une projection de fond du signal de fond, la projection d'artefact et respectivement la projection de fond étant une projection du signal d'artefact respectivement du signal de fond sur la base de la transformée en ondelettes discrète,
- pour chaque niveau m de la transformation, la détermination d'une fonction de densité cumulative de l'artefact

$$F_m^{(art)}(x) = \frac{1}{N_m} \sum_{n=1}^{N_m} 1_{\left|w_{m,n}^{(art)}\right| < x}$$ où $N_m$ est le nombre total de coefficients de la projection de l'artefact au niveau m, $\left|w_{m,n}^{(art)}\right|$ est la valeur absolue d'un n-ième coefficient de la projection de l'artefact au niveau m, et

$1_{\left|w_{m,n}^{(art)}\right| < x}$ est une fonction indicatrice qui renvoie la valeur 1 si $\left|w_{m,n}^{(art)}\right| < x$ et sinon la valeur 0,

- pour chaque niveau m de la transformation, la détermination d'une fonction de densité cumulative au sol

$$F_m^{(ref)}(x) = \frac{1}{N_m} \sum_{n=1}^{N_m} 1_{\left|w_{m,n}^{(ref)}\right| < x}$$ où $N_m$ est le nombre total de coefficients de la projection de l'artefact au niveau m, $\left|w_{m,n}^{(ref)}\right|$ est la valeur absolue d'un n-ième coefficient de la projection de l'artefact au niveau m, et $1_{\left|w_{m,n}^{(ref)}\right| < x}$ est une fonction indicatrice qui renvoie la valeur 1 si $\left|w_{m,n}^{(ref)}\right| < x$ et sinon la valeur 0,

- pour chaque niveau m et pour chaque coefficient d'artefact $w_{m,n}^{(art)}$, la détermination d'un coefficient corrigé $w_{m,n}^{(corr)}$ d'une projection corrigée, le coefficient d'artefact et le coefficient corrigé étant indexés de manière similaire, en suivant les sous-étapes :

-- la détermination de la valeur $F_m^{(art)}\left(\left|w_{m,n}^{(art)}\right|\right)$,

-- la détermination d'une valeur intermédiaire x vérifiant $F_m^{(ref)}(x) = F_m^{(art)}\left(\left|w_{m,n}^{(art)}\right|\right)$,

- l'établissement du coefficient corrigé $w_{m,n}^{(corr)}$ égal à $w_{m,n}^{(art)} \times min\left\{1, \frac{x}{\left|w_{m,n}^{(art)}\right|}\right\}$,

- la détermination d'un signal corrigé à l'aide de la transformée en ondelettes discrète sur la base de la projection corrigée,
- le remplacement, dans le signal EEG reçu, du signal artéfact par le signal corrigé.

7. Procédé selon l'une quelconque des revendications 1 à 6, comprenant une détermination d'une quantité minimale d'anesthésique à injecter pour que le sujet (2) reste en anesthésie générale ou en sédation, la détermination étant sur la base du premier paramètre.

8. Dispositif d'assistance à l'anesthésie générale ou à la sédation (1) comprenant un processeur (3) configuré pour mettre en œuvre le procédé mis en œuvre par ordinateur selon les revendications 1 à 7.

9. Support lisible par ordinateur comprenant des instructions exécutables par ordinateur qui, lorsqu'elles sont exécutées par un ordinateur, amènent l'ordinateur à réaliser le procédé selon l'une quelconque des revendications 1 à 7.

**Fig. 1**

**Fig. 2**

**Fig. 3**

**Fig. 4**

Fig. 5

Fig. 6

**Fig. 7**

Fig. 8

$t_{ref}$

Induction phase

$t_{ind}$

Maintenance

$t_{fin}$

31 — Checkpoint 1 $P(S=1 \mid t_{\alpha S})$

32 — Checkpoint 1 $P(S=1 \mid t_{IES})$

34 — Checkpoint 2 $P(S=1 \mid t_{\alpha S}, a)$

35 — Checkpoint 2 $P(S=1 \mid t_{\alpha S}, t_{IES}, a)$

36 — Checkpoint 2 $P(S=1 \mid t_{IES}, t_{\alpha S}, a)$

33 — Checkpoint 2 $P(S=1 \mid a)$

37 — Checkpoint 3 $P(S=1 \mid t_{\alpha S}, a, t_{IES})$

38 — Checkpoint 3 $P(S=1 \mid a, t_{\alpha S}, t_{IES})$

**Fig. 9**

**Fig. 10**

**Fig. 11**

EP 4 376 703 B1

Fig. 12

**EP 4 376 703 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021081504 A1